(19) Europäisches Patentamt
European Patent Office
Office européen des brevets

(11) Veröffentlichungsnummer: 0 004 529
B1

(12) EUROPÄISCHE PATENTSCHRIFT

(45) Veröffentlichungstag der Patentschrift: 17.06.81

(51) Int. Cl.³: C 07 D 233/50, A 61 K 31/415

(21) Anmeldenummer: 78101569.8

(22) Anmeldetag: 05.12.78

(54) **Tautomere Arylaminoimidazolinderivate, deren Herstellung und sie enthaltende Arzneimittel zur Bekämpfung von Schmerzzuständen.**

(30) Priorität: 17.03.78 DE 2811847

(43) Veröffentlichungstag der Anmeldung: 17.10.79 Patentblatt 79/21

(45) Bekanntmachung des Hinweises auf die Patenterteilung: 17.06.81 Patentblatt 81/24

(84) Benannte Vertragsstaaten: BE CH DE FR GB IT NL SE

(56) Entgegenhaltungen:
DE - A - 2 521 709
NL - A - 76 08 971

(73) Patentinhaber: Lentia Gesellschaft mit beschränkter Haftung
Schwanthalerstrasse 39 Postfach 20 16 26
D-8000 München 2 (DE)

(72) Erfinder: Franzmair, Rudolf, Dr.
Franz-Xaver-Müllerweg 9
A-4033 Linz-Ebelsberg (AT)
Erfinder: Enzenhofer, Rita, Dr.
Hammerweg 78
A-4050 Traun (AT)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Courier Press, Leamington Spa, England.

# Tautomere Arylaminoimidazolinderivate, deren Herstellung und sie enthaltende Arzneimittel zur Bekämpfung von Schmerzzuständen

Es ist bekannt, daß Arylaminoimidazoline, insbesondere das 2,6-Dichlorphenylamino-2-imidazolin (Clonidin) eine ausgeprägte, hypotensive Wirkung besitzen, die mit einer sedativen Wirking gepaart ist. Daneben besitzen einige dieser Verbindungen auch eine mehr oder weniger ausgeprägte analgetische Wirkung, die aber wegen des gleichzeitigen Vorliegens der hypotensiven und zentraldämpfenden Wirkung als nicht auswertbar gilt. (Siehe dazu die Arbeit von R. D. E. Sewell und P. S. J. Spencer, Progress in Medicinal Chemistry, 14, 1977, Seite 254.) Dort ist auch beschrieben, daß bestimmte Derivate des Phenylaminoimidazolins, z.B. das am Anilin-N durch eine Allylgruppe substituierte Clonidin, ein günstigeres Verhältnis von analgetischer zu hypotensiver Wirkung zeigen als Clonidin selbst, doch sind auch hier die anderen pharmakologischen Wirkungen noch zu stark ausgeprägt. Eine hypotensive Wirkung ist auch für Benzoylderivate von Arylaminoimidazolin speziell für die Verbindung 1-Benzoyl-2-(2′,6′-dichlorphenylamino)-2-imidazolin in der AT—PS Nr. 330 769 beschreiben worden, wobei bei dieser Verbindung die dämpfende Wirkung auf das zentrale Nervensystem und damit die sedative Wirkung wesentlich weniger ausgeprägt ist.

Überraschenderweise wurde gefunden, daß Arylaminoimidazolinderivate der beiden tautomeren Formeln

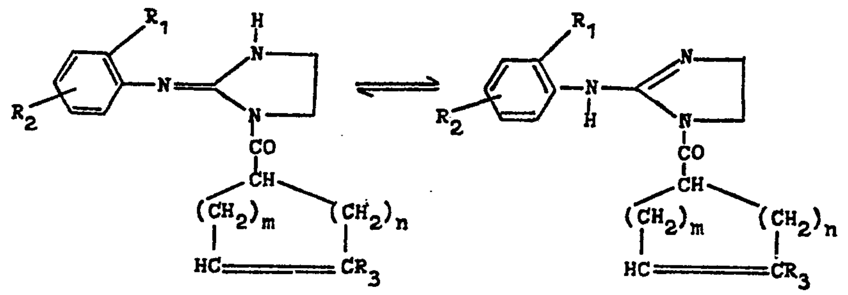

(I)

in denen $R_1$ und $R_2$ Halogenatome oder eine Alkylgruppe mit 1 bis 4 C-Atomen und $R_3$ ein Wasserstoffatom oder einen Alkylrest mit 1 bis 4 C-Atomen bedeuten und n und m je eine ganze Zahl von 1 bis 3 darstellen, mit der Maßgabe, daß die Summe n + m eine Zahl von 2 bis einschließlich 5 ist und die Säureadditionssalze dieser Verbindungen eine ausgeprägte analgetische Wirkung besitzen, während eine Wirkung auf den Blutdruck, sei es eine diesen senkende oder steigernde, bzw. eine Wirkung auf das zentrale Nervensystem praktisch nicht in Erscheinung tritt. Dies ist umso überraschender, da sich ja die Verbindungen der Formel I, vor allem jene, in denen n + m die Zahl 3 bedeutet, sodaß sie Cyclohexenoylderivate sind, von den Benzoylderivaten gemäß AT—PS Nr. 330 769 nur durch den Sättigungsgrad des sechsgliedrigen Ringes des Acylrestes unterscheiden, bei welchen die blutdrucksenkende Wirkung besonders ausgeprägt ist, die zentraldämpfende Wirkung sehr gering ist und das Verhältnis analgetischer zu hypotensiver Wirkung stark zu Gunsten der hypotensiven verschoben ist.

Die Verbindungen der Formel I können daher vorteilhaft zur Behandlung von Schmerzzuständen aller Art eingesetzt werden, wobei sie entweder als Basen oder aber auch in Form der Säureadditionssalze oral, enteral oder parenteral verabreicht werden können. Sie können natürlich mit anderen Wirkstoffen, z.B. Spasmolytika und Tranquilizer kombiniert werden.

Gute Wirkungen zeigen Verbindungen der Formel I mit 2 Cl-Atomen in Phenylkern.

Besonders bevorzugt sind unter den Verbindungen der Formel I vor allem solche, die in 2- und 6-Stellung des Anilinrestes substituiert sind, wobei wiederum die 2,6-Dichlorverbindungen und die 2-Chlor-6-methylverbindungen bevorzugt sind. Unter den Acylresten sind besonders die Cyclohex-3-enoylreste zu nennen, aber auch Verbindungen der Formel I, die Cyclopent-3-enoyl- und Cyclohept-4-enoylreste tragen, zeigen günstige Wirkungen. Verbindungen mit sehr starker, analgetischer Wirkung und nur sehr geringen, für die praktische Anwendung vernachlässigbaren Wirkungen auf Blutdruck und auf das zentrale Nervensystem sind beispielsweise 1-(Cyclohex-3″-en-1″-oyl)-2-(2′,6′-dichlorphenylamino)-2-imidazolin, 1-(Cyclohex-3″-en-1″-oyl)-2-(2′-chlor-6′-methylphenylamino)-2-imidazolin, 1-(Cyclohex-3″-en-1″-oyl)-2-(2′,3′-dichlorphenylamino)-2-imidazolin, 1-(Cyclohex-3″-en-1″-oyl)-2-(2′-methyl-3′-chlorphenylamino)-2-imidazolin, 1-(4″-Methyl-cyclohex-3″-en-1″-oyl)-2-(2′,6′-dichlorphenylamino)-2-imidazolin, 1-(Cyclohept-4″-en-1″-oyl)-2-(2′,6′-dichlorphenylamino)-2-imidazolin und 1-(Cyclopent-3″-en-1″-oyl)-2-(2′,6′-dichlorphenylamino)-2-imidazolin.

Die Herstellung der Verbindungen der Formel I gelingt, indem man

a) ein Anilinderivat der allgemeinen Formel

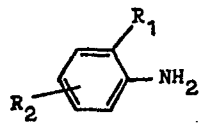

(II)

in der $R_1$ und $R_2$ wie oben definiert sind, mit einem 1-Acylimidazolidin-2-on der allgemeinen Formel

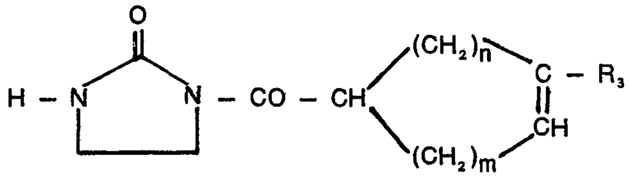

(III)

in der $R_3$, n und m wie in Formel I definiert sind, in Gegenwart von mindestens 2 Äquivalenten Phosphoroxychlorid umsetzt und das entstandene Reaktionsprodukt nach Abtrennen des überschüssigen Phosphoroxychlorids milde hydrolysiert oder

b) 2-Arylamino-2-imidazoline der allgemeinen Formeln

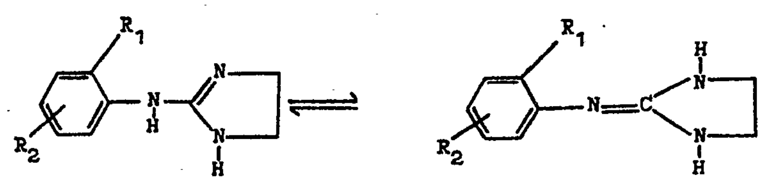

(IV)

in denen $R_1$ und $R_2$ wie in Patentanspruch 1 definiert sind, mit einem Carbonsäurederivat der Formel

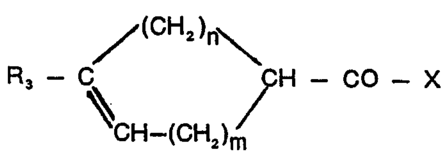

(V)

in der $R_3$, n und m wie oben angegeben definiert sind und X einen über ein Stickstoffatom an die Carbonylgruppe gebundenen Rest eines heteroaromatischen fünfgliederigen Ringes mit mindestens zwei Stickstoffatomen, der gegebenenfalls mit einem Benzolring kondensiert sein kann, einen Rest der Formel

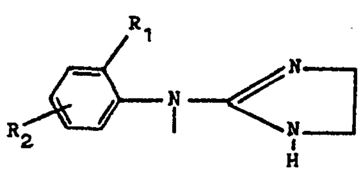

(VI)

in der $R_1$ und $R_2$ wie oben definiert sind oder einen Säurerest der Formel

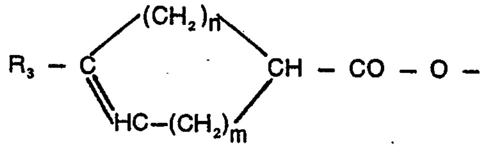

(VII)

in der $R_3$, n und m wie oben definiert sind, darstellt, umsetzt und die erhaltenen Verbindungen der Formel I als freie Basen oder als Säureadditionssalze isoliert.

Die Umsetzung der Aniline der Formel II mit dem 1-Acylimidazolidin-2-on der Formel III kann in inerten, organischen Lösungsmitteln, wie halogenierten Kohlenwasserstoffen, bevorzugt in Phosphoroxychlorid selbst, bei Temperaturen zwischen Raumtemperatur und dem Siedepunkt des gewählten Lösungsmittels durchgeführt werden. Bevorzugt ist hierbei eine Reaktionstemperatur von 50 bis 80°C.

Das im Überschuß vorliegende Phosphoroxychlorid wird nach der Reaktion im Vakuum abdestilliert und kann ohne weitere Reinigung wieder erneut eingesetzt werden.

Der Rückstand nach dem Entfernen des Phosphoroxychlorids stellt ein phosphorhältiges Zwischenprodukt dar, das durch milde Hydrolyse unter Bildung des gewünschten Produktes der Formel I gespalten wird. Diese milde Hydrolyse kann bevorzugt so vorgenommen werden, daß der Eindampfrückstand in einem inerten, mit Wasser nicht mischbaren Lösungsmittel, beispielsweise in Methylenchlorid, Chloroform, Tetrachlorkohlenstoff, aromatische Kohlenwasserstoffe oder Äther aufgenommen wird. Durch Zusatz von Eiswasser wird das P-hältige Zwischenprodukt zersetzt, worauf die wäßrige, saure Phase durch Zugabe einer Base, z.B. von Alkalimetallcarbonaten, Alkalimetallbicarbonaten, Natronlauge, Kalilauge oder Ammoniak neutralisiert wird. Hierbei ist darauf zu achten, daß eine stärkere alkalische Reaktion nicht erreicht wird. Vorzugsweise soll der pH-Wert der wäßrigen Phase zwischen 7 und 8 liegen. Das gewünschte Endprodukt der Formel I kann dann aus der organischen Phase durch Eindampfen isoliert werden. Es ist aber ebensogut möglich, ohne organisches Lösungsmittel zu arbeiten. In diesem Fall wird der Eindampfrückstand direkt mit Eiswasser zersetzt, worauf zur Gewinnung der Base die entstandene saure Lösung neutralisiert bzw. ganz schwach alkalisch gemacht wird. Diese Vorgangsweise empfiehlt sich vor allem dann, wenn die herzustellende Verbindung der Formel I aus der wäßrigen Phase auszukristallisieren imstande ist.

Die Ausgangsmaterialien der Formel II werden durch Umsetzung von Imidazolidin-2-on mit dem entsprechenden Carbonsäurechlorid in einem polaren Lösungsmittel, z.B. Acetonitril, erhalten.

Wird zur Herstellung der Verbindungen der Formel I von Verbindungen der Formel IV ausgegangen, so wird die Reaktion mit den Verbindungen der Formel V vorzugsweise in organischem Medium ausgeführt, wobei sowohl polare als auch apolare Lösungsmittel Verwendung finden können. Als solche Lösungsmittel können beispielsweise aromatische Kohlenwasserstoffe, wie Benzol, Toluol oder Xylol, Äther, wie Tetrahydrofuran, Di-äthyläther und Dioxan, Ester, wie Athylacetat, halogenierte Kohlenwasserstoffe, wie Methylenchlorid, Chloroform oder Tetrachlorkohlenstoff, Ketone, zum Beispiel Aceton, Methyläthylketon, sowie aprotische, polare Lösungsmittel, wie beispielsweise Acetonitril, Dimethylformamid oder Dimethylsulfoxyd, dienen.

Die Verbindungen der Formel V können sowohl aktive Amide oder auch Anhydride sein. Unter den aktiven Amiden sind vor allem Azolide brauchbar, die sich von Imidazol, 1,2,4-Triazol, Tetrazol, Benzimidazol, Benztriazol ableiten. X in Formel V kann aber auch die Gruppe

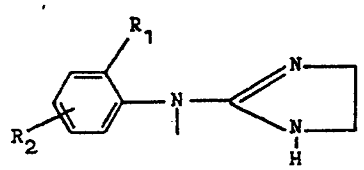

. (VI)

bedeuten. Sind in dieser Formel VI und in der zu acylierenden Verbindung der Formel IV die Substituenten $R_1$ und $R_2$ des Benzolkerns gleich, so ist es nicht nötig, die Verbindung der Formel IV in äquimolaren Mengen oder einem nur geringen Unterschuß zur eingesetzten Verbindung der Formel V einzusetzen. Man kann hier vielmehr mit einem wesentlichen Unterschuß an Verbindung der Formel IV, zweckmäßig unter einem halben Äquivalent, vorzugsweise sogar nur mit einem Zehntel Äquivalent, bezogen auf das aktive Amid der Formel V, das Auslangen finden, da ja im Laufe der Acylierung laufend die Verbindung der Formel IV aus dem aktiven Amid der Formel V in Freiheit gesetzt wird. Diese Variante des erfindungsgemäßen Verfahrens wird bevorzugt durch Kochen der Ausgangsmaterialien in einem aprotischen, inerten Lösungsmittel, z.B. Toluol oder Xylol durchgeführt.

Ist die Verbindung der Formel V ein Säureanhydrid, so kann nicht nur in organischem Medium, sondern auch in wäßrigem, schwach alkalischem Medium gearbeitet werden. Besonders vorteilhaft ist hier die Umsetzung bei Raumtemperatur.

Wird in wasserfreiem Medium, z.B. in wasserfreiem Tetrahydrofuran gearbeitet, hat sich der Zusatz eines säurebindenden Mittels, z.B. eines Amins, wie Triäthylamin, als günstig erwiesen.

Die Aufarbeitung gestaltet sich bei allen Varianten zur Herstellung der erfindungsgemäßen Verbindungen, die von den Verbindungen der Formel IV ausgehen, sehr einfach. Der nach Abdampfen des Lösungsmittels verbleibende Eindampfrückstand kann einfach durch Umkristallisieren gereinigt werden. In einigen Fällen empfiehlt es sich, den Trockenrückstand zunächst mit Wasser zu digerieren, worauf Kristallisation eintritt.

Wird die Umsetzung mit Verbindungen der Formel V vorgenommen, in denen X den Rest eines heteroaromatischen, fünfgliedrigen Ringes mit mindestens zwei N-Atomen, der mit Benzol kondensiert sein kann, darstellt, also mit einem Azolid vorgenommen, so ist es nicht in allen Fällen nötig, dieses Azolid der Formel V in einem getrennten Arbeitsgang herzustellen. Azolide der Formel V können auch in situ aus dem entsprechenden Azol und einem Säurechlorid der Formel

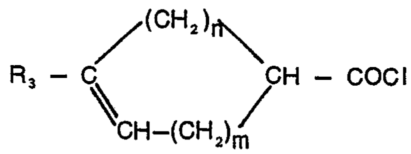

(VIII)

erzeugt und direkt weiterverwendet werden. So ist es z.B. möglich, Säurechloride der Formel VIII in Tetrahydrofuran mit Imidazol umzusetzen und nach beendeter Reaktion und Abfiltrieren des Azolhydrochlorids in die Reaktionslösung eine Lösung der Verbindung der Formel IV einzutragen. Eine in situ-Darstellung eines Azolids der Formel V gelingt auch durch Umsetzung der Säure der Formel

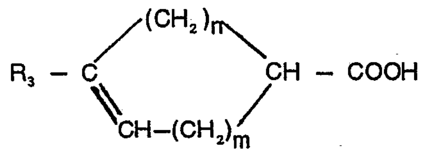

(IX)

mit N,N'-Carbonyldiimidazol.

Aktive Amide, in denen X die Gruppe der Formel VI darstellt, können durch Umsetzung von Verbindungen der Formel IV mit den Säurechloriden der Formel VIII erhalten werden.

Die Verbindungen der Formel I können als freie Basen oder nach Überführung in Säureadditionssalze solche isoliert werden. Geeignet sind hierbei vor allem Salze starker Mineralsäuren, wie Hydrohalogenide, Sulfate, Cyclohexylsulfamate usw.

Die Verbindungen der Formel I sowie deren Säureadditionssalze können oral, enteral oder auch parenteral angewandt werden. Sie können auch mit anderen Wirkstoffen, wie andere Analgetika, Spasmolytika, Tranquilizer usw. kombiniert verabreicht werden. Geeignete galenische Darreichungsformen sind beispielsweise Tabletten, Dragees, Kapseln, Zäpfchen, Lösungen, Emulsionen oder Pulver; hierbei können zu deren Herstellung die üblicherweise verwendeten galenischen Hilfs-, Träger-, Spreng- oder Schmiermittel bzw. Substanzen zur Erzielung einer Depotwirkung Anwendung finden. Die Herstellung derartiger galenischer Darreichungsformen erfolgt auf herkömmliche Weise nach bekannten Methoden.

Die folgenden Beispiele erläutern die Erfindung.

Beispiel 1

8,1 g (50 mmol) 2,6-Dichloranilin, 10,67 g (55 mmol) 1-Cyclohex-3'-en-1'-oyl-imidazolidin-2-on und 100 ml Phosphoroxychlorid werden 70 Stunden bei 50°C gerührt. Im Vakuum wird das überschüssige Phosphoroxychlorid entfernt, der Rückstand mit etwa 500 ml Eiswasser versetzt, und eine Stunde bei Raumtemperatur gerührt. Dann wird solange eine gesättigte Kaliumbicarbonatlösung zugegeben, bis ein pH = 7 bestehen bleibt, wobei Kristallabscheidung eintritt. Es wird filtriert und das Kristallisat getrocknet. Man erhält 16,7 g (98,8% der Theorie) rohes 1-(Cyclohex-3''-en-1''-oyl)-2-(2',6'-dichlorphenylamino)-2-imidazolin. Es wird aus Isopropanol umkristallisiert und man erhält 13,77 g (81,5% der Theorie) analysenreines Produkt vom Fp = 159—162°C.

5 g des so erhaltenen 1-(Cyclohex-3''-en-1''-oyl)-2-(2',6'-dichlorphenylamino)-2-imidazolin werden in 50 ml wasserfreiem Methylenchlorid gelöst und mit etwa 4 ml ca. 20%iger ätherischer Salzsäure versetzt. Aus der zunächst klaren Lösung scheiden sich nach etwa zehn Minuten Kristalle ab. Es wird noch eine Stunde bei 0°C gehalten, 50 ml Äther zugesetzt, filtriert, mit Äther gewaschen und getrocknet. Man erhält so 5,10 g (92,1% der Theorie) 1-(Cyclohex-3''-en-1''-oyl)-2-(2',6'-dichlorphenylamino)-2-imidazolinhydrochlorid, $FP_1$ = 180—187°C, $FP_2$ = 253—258°C unter Zersetzung.

Das Ausgangsmaterial wird hergestellt, indem man Imidazolidin-2-on und Cyclohex-3-en-1-carbonsäurechlorid im Molverhältnis 2:1 in Acetonitril bei Raumtemperatur umsetzt. Man erhält 1-(Cyclohex-3'-en-1'-oyl)-imidazolidin-2-on in einer Ausbeute von 85,8% der Theorie. FP = 119—123°C.

Beispiel 2

1,62 g (10 mmol) 2,6-Dichloroanilin, 1,80 g (10 mmol) 1-(Cyclopent-3'-en-1'-oyl)-imidazolidin-2-on und 20 ml Phosphoroxychlorid werden 20 Stunden bei 80°C gerührt. Das überschüssige Phosphoroxychlorid wird im Vakuum entfernt, der teilweise kristalline Rückstand wird in 50 ml Methylenchlorid aufgenommen, 50 ml Eiswasser zugesetzt und unter heftigem Rühren wird mit gesättigter Sodalösung versetzt bis ein pH von etwa 7,5 bis 8, bestehen bleibt. Die organische Phase wird abgetrennt, die wäßrige Phase noch drei Mal mit je 7 ml Methylenchlorid extrahiert, die Methylenchloridphasen vereinigt, mit Wasser neutral gewaschen, über Natriumsulfat getrocknet und eingedampft. Der Rückstand wird aus Isopropanol umkristallisiert, wobei man 2,35 g (72,5% der Theorie) 1-(Cyclopent-3''-en-1''-oyl)-2-(2',6'-dichlophenylamino)-2-imidazolin, Fp = 170—172°C, erhält.

Das Ausgangsmaterial wird hergestellt, indem man Imidazolidin-2-on und Cyclopent-3-en-1-carbonsäurechlorid im Molverhältnis 2:1 in Acetonitril bei Raumtemperatur umsetzt. Man erhält 1-(Cyclopent-3'-en-1'-oyl)-imidazolidin-2-on in einer Ausbeute von 66,7% der Theorie. Fp = 166—169°C.

Beispiel 3

0,94 g (5,8 mmol) 2,6-Dichloranilin, 1,20 g 1-(Cyclohept-4'-en-1'-oyl)-imidazolidin-2-on und 30 ml Phosphoroxychlorid werden 40 Stunden bei 70°C gerührt.

In Vakuum wird das überschüssige Phosphoroxychlorid entfernt, der Rückstand wird in Toluol suspendiert, mit Eiswasser versetzt und wie in Beispiel 2 beschrieben aufgearbeitet.

Man erhält 2,0 g kristallines Rohprodukt, das nach Umkristallisation aus Isopropanol 1,60 g (78,43% der Theorie) 1-(Cyclohept-4''-en-1''-oyl)-2-(2',6'dichlorphenylamino)-2-imidazolin, Fp = 161—163°C, ergibt.

Das Ausgangsmaterial wird erhalten, indem 2 Äquivalente Imidazolidin-2-on in absolutem Acetonitril suspendiert werden und dazu unter Rühren bei Raumtemperatur 1 Äquivalent Cyclohept-4-en-1-carbonsäurechlorid zugetropft wird. Der Ansatz wird 18 Stunden bei Raumtemperatur gerührt, das Acetonitril im Vakuum entfernt, der Rückstand in Wasser digeriert, filtriert, getrocknet und aus Cyclohexan:Isopropanol (4:1) umkristallisiert. Man erhält in 75%iger Ausbeute 1-(Cyclohept-4'-en-1'-oyl)-imidazolidin-2-on, Fp = 122—125°C.

Beispiel 4

4,26 g (30 mmol 2-Chlor-6-methylanilin, 6,40 g (33 mmol) 1-(Cyclohex-3'-en-1'-oyl)-imidazolidin-2-on und 60 ml Phosphoroxychlorid werden 69 Stunden bei 60°C gerührt. Es wird wie in Beispiel 2 beschrieben, aufgearbeitet. Der nach dem Eindampfen der Methylenchloridphasen erhaltene, ölige Rückstand wird mit 100 ml 50%igen Acetonitrol verreiben und einige Zeit bei Raumtemperatur belassen, wobei Kristallisation eintritt. Es wird filtriert, getrocknet und aus Cyclohexan umkristallisiert. Man erhält so 7,87 g (82,5% der Theorie) 1-(Cyclohex-3''-en-1''-oyl)-2-(2'-chlor-6'-methylphenylamino)-2-imidazolin, Fp = 112—114°C.

Beispiel 5

3,57 g (22 mmol) 2,6-Dichloranilin, 4,90 g (23,5 mmol) 1-(Cyclooct-4'-en-1'-oyl)-imidazolidin-2-on und 100 ml Phosphoroxychlorid werden 20 Stunden bei 90°C gerührt und wie in Beispiel 2 beschrieben, aufgearbeitet und aus Isopropanol über Aktivkohle umkristallisiert. Man erhält so 3,01 g (37,17% der Theorie) 1-(Cyclooct-4'-en-1''-oyl)-2-(2',6'-dichlorphenylamino)-2-imidazolin, Fp = 136—138°C.

Das Ausgangsmaterial wird erhalten, indem in absolutem Acetonitril bei Raumtemperatur Imidazolidin-2-on und Cyclooct-4-en-1-säurechlorid im Molverhältnis 2:1 umgesetzt werden. 1-(Cyclooct-4'-en-1'-oyl)-imidazolidin-2-on stellt ein zähes, nicht kristallisierendes Öl dar. Seine Spektren (IR, UV, NMR) stehen mit der angegebenen Struktur im Einklang.

Beispiel 6

1,55 g (22 mmol) Imidazol werden in 50 ml absolutem Tetrahydrofuran gelöst, und dazu unter Rühren eine Lösung von 1,57 g (11 mmol) Cyclohex-3-en-1-carbonsäurechlorid in 10 ml absolutem Tetrahydrofuran getropft und 4 Stunden bei Raumtemperatur gerührt. Vom ausgefallenen Imidazolhydrochlorid wird abfiltriert und dieses mit 5 ml absolutem Tetrahydrofuran gewaschen. Zum Filtrat wird eine Lösung vom 2,30 g (10 mmol) 2-(2',6'-Dichlorphenylamino)-2-imidazolin in 15 ml absolutem Tetrahydrofuran gefügt, und 20 Stunden bei Raumtemperatur belassen. Dann wird im Vakuum eingedampft und der ölige Rückstand mit ca. 50 ml Wasser verrieben, wobei Kristallisation eintritt. Es wird filtriert, mit Wasser gewaschen, getrocknet und aus Isopropanol umkristallisiert, wobei man 3,10 g (91,7% der Theorie) 1-Cyclohex-3''-en-1''-oyl)-2-(2',6'-dichlorphenylamino)-2-imidazolin, Fp = 159—162°C, erhält.

Beispiel 7

1,69 g (5 mmol) 2-[N-(Cyclohex-3''-en-1''-oyl)-N-(2',6'-dichlorphenyl)-amino]-2-imidazolin und 115 mg (0,5 mmol) 2-(2',6'-Dichlorphenylamino)-2-imidazolin werden in 50 ml absolutem Toluol 144 Stunden am Rückfluß erhitzt. Dann wird im Vakuum zur Trockne eingedampft und der Rückstand aus Isopropanol umkristallisiert, wobei man 1,306 g (77,2% der Theorie) 1-(Cyclohex-3''-en-1''-oyl)-2-(2',6'-dichlorphenylamino)-2-imidazolin, Fp = 159—162°C, erhält.

Das Ausgangsmaterial wurde wie folgt hergestellt:

0,54 g (2 mmol) Tetra-n-butylammoniumchlorid werden in 30 ml Wasser gelöst, 40 ml 1 n Natronlauge zugesetzt und mit einer Lösung von 9,20 g (40 mmol) 2-(2',6'-Dichlorphenylamino)-2-imidazolin in 40 ml Methylenchlorid versetzt. Das Gemisch wird auf 0°C gekühlt und unter heftigem Rühren eine Lösung von 5,73 g (40 mmol) Cyclohex-3-en-1-carbonsäurechlorid in 30 ml Methylenchlorid zugetropft und nach be endetem Zutropfen noch 4 Stunden bei 0°C gerührt. Dabei fällt ein Kristallisat aus, das abfiltriert, mit Methylenchlorid/Wasser gewaschen und getrocknet wird. Man erhält so 7,75 g erstes Kristallisat.

Vom Filtrat werden die Phasen getrennt, die wäßrige Phase noch 2 Mal mit Methylenchlorid

extrahiert, die Methylenchloridphasen vereinigt, mit Wasser gewaschen, über Natriumsulfat getrocknet und eingedampft. Man erhält 5,70 g zweites Kristallisat, das mit dem ersten vereinigt aus Isopropanol umkristallisiert wird. Man erhält 10,80 g (80,0% der Theorie) 2-[N-(Cyclohex-3″-en-1″-oyl)-N-(2′,6′-dichlorphenyl)-amino]-2-imidazolin, Fp = 218—220°C.

Beispiel 8

1,39 g (11 mmol) Cyclohex-3-en-1-carbonsäure werden in 40 ml absolutem Benzol gelöst, 1,79 g (11 mmol) N,N′-Carbonyldiimidazol zugefügt und 1 Stunde bei Raumtemperatur belassen. Dann wird eine Lösung von 2,10 g (10 mmol) 2-(2′-Chlor-6′-methylphenylamino)-2-imidazolin in 30 ml absolutem Benzol zugesetzt und 45 Minuten unter Rückfluß gekocht. Das Benzol wird im Vakuum entfernt und der ölige Rückstand mit Wasser digeriert, wobei Kristallisation eintritt. Es wird filtriert, mit Wasser gewaschen, getrocknet und das Cyclohexan umkristallisiert, wobei man 2,62 g (82,44% der Theorie) 1-Cyclohex-3″-en-1″-oyl)-2-(2′-chlor-6′-methylphenylamino)-2-imidazolin, Fp = 112—114°C, erhält.

Beispiel 9

2,30 g (10 mmol) 2-(2′,6′-Dichlorphenylamino)-2-imidazolin werden in 100 ml wasserfreiem Tetrahydrofuran gelöst, 1,01 g (10 mmol) wasserfreies Triäthylamin zugefügt und dazu unter Rühren bei Raumtemperatur langsam eine Lösung von 2,34 g (10 mmol) Cyclohex-3-en-1-carbonsäureanhydrid ($KP_{0.1}$ = 126—127°C) in 50 ml wasserfreiem Tetrahydrofuran zugetropft und über Nacht bei Raumtemperatur weiter gerührt. Die klare Lösung wird im Vakuum eingedampft, der Rückstand in etwa 50 ml Methylenchlorid aufgenommen, zwei Mal je 20 ml 3%iger Natriumbicarbonatlösung und zwei Mal mit Wasser gewaschen, über Natriumsulfat getrocknet und eingedampft. Der kristalline Rückstand wird aus Isopropanol umkristallisiert, wobei man 3,12 g (92,3% der Theorie) 1-(Cyclohex-3″-en-1″-oyl)-2-(2′,6′-dichlorphenylamino)-2-imidazolin, Fp = 159—162°C, erhält.

In analoger Weise zu den Beispielen 1 bis 9 werden erhalten:

1-(4″-Methyl-cyclohex-3″-en-1″-oyl)-2-(2′,6′-dichlorphenylamino)-2-imidazolin, Fp = 152—154°C
1-(Cyclohex-3″-en-1″-oyl)-2-(2′,3′-dichlorphenylamino)-2-imidazolin, Fp = 147—149°C
1-(Cyclohex-3″-en-1″-oyl)-2-(2′-methyl-3′-chlor-phenylamino)-2-imidazolin, Fp = 112—115°C
1-(Cyclohex-3″-en-1″-oyl)-2-(2′-chlor-4′-methyl-phenylamino)-2-imidazolin, Fp = 124—126°C.

**Patentansprüche**

1. Tautomere Arylaminoimidazolinderivate der allgemeinen Formeln

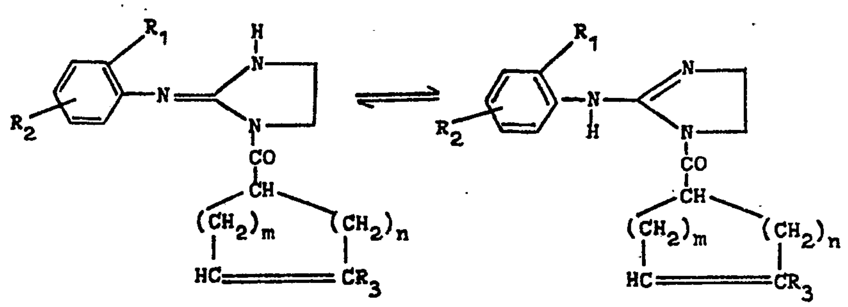

(I)

in deren $R_1$ und $R_2$ Halogenatome oder eine Alkylgruppe mit 1 bis 4 C-Atomen und $R_3$ ein Wasserstoffatom oder einen Alkylrest mit 1 bis 4 C-Atomen bedeuten und n und m je eine ganze Zahl von 1 bis 3 darstellen, mit der Maßgabe, daß die Summe n+m eine Zahl von 2 bis einschließlich 5 ist und die Säureadditionssalze dieser Verbindungen.

2. Arylaminoimidazolinderivat nach Patentanspruch 1, welches 1-(Cyclohex-3″-en-1″-oyl)-2-(2′,6′-dichlorphenylamino)-2-imidazolin ist.

3. Arylaminoimidazolinderivat nach Patentanspruch 1, welches 1-(Cyclohex-3″-en-1″-oyl)-2-(2′-chlor-6′-methyl-phenylamino)-2-imidazolin ist.

4. Arylaminoimidazolinderivat nach Patentanspruch 1, welches 1-(Cyclohex-3″-en-1″-oyl)-2-(2′,3′-dichlorphenylamino)-2-imidazolin ist.

5. Arylaminoimidazolinderivat nach Patentanspruch 1, welches 1-(Cyclohex-3″-en-1″-oyl)-2-(2′-methyl-3′-chlorphenylamino)-2-imidazolin ist,

6. Arylaminoimidazolinderivat nach Patentanspruch 1, welches 1-(4″-Methyl-cyclohex-3″-en-1″-oyl)-2-(2′,6′-dichlorphenylamino)-2-imidazolin ist.

7. Arylaminoimidazolinderivat nach Patentanspruch 1, welches 1-(Cyclohept-4″-en-1″-oyl)-2-(2′,6′-dichlorphenylamino)-2-imidazolin ist.

8. Arylaminoimidazolinderivat nach Patentanspruch 1, welches 1-(Cyclopent-3″-en-1″-oyl)-2-(2′,6′-dichlorphenylamino)-2-imidazolin ist.

9. Verfahren zur Herstellung von Verbindungen gemäß den Patentansprüchen 1 bis 8, dadurch gekennzeichnet, daß man
a) ein Anilinderivat der allgemeinen Formel

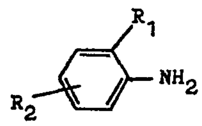

(II)

in der $R_1$ und $R_2$ wie in Patentanspruch 1 definiert sind, mit einem 1-Acyl-imidazolidin-2-on der allgemeinen Formel

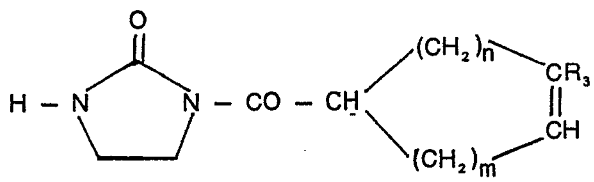

(III)

in der $R_3$, n und m wie in Patentanspruch 1 definiert sind, in Gegenwart von mindestens 2 Äquivalenten Phosphoroxychlorid umsetzt und das entstandene Reaktionsprodukt nach Abtrennen des überschüssigen Phosphoroxychlorid milde hydrolysiert oder

b) 2-Arylamino-2-imidazoline der allgemeinen Formeln

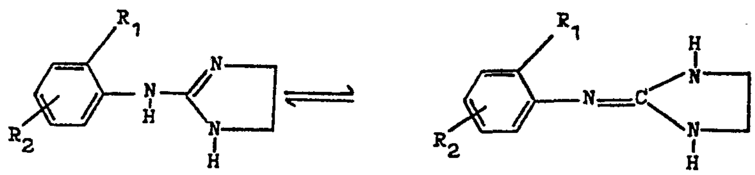

(IV)

in denen $R_1$ und $R_2$ wie in Patentanspruch 1 definiert sind, mit einem Carbonsäurederivat der Formel

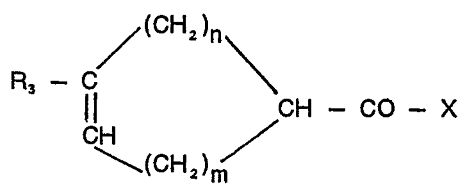

(V)

in der $R_3$, n und m wie in Patentanspruch 1 definiert sind und X einen über ein Stickstoffatom an die Carbonylgruppe gebundenen Rest eines heteroaromatischen fünfgliederigen Ringes mit mindestens zwei Stickstoffatomen, der gegebenenfalls mit einem Benzolkern kondensiert sein kann, einen Rest der Formel

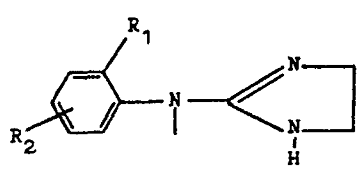

(VI)

in der $R_1$ und $R_2$ wie in Patentanspruch 1 definiert sind oder einen Säurerest der Formel

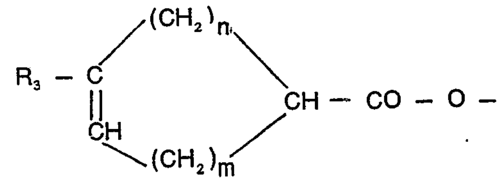

(VII)

in der $R_3$, n und m wie in Patentanspruch 1 definiert sind, darstellt, umsetzt und die erhaltenen Verbindungen der Formel I als freie Basen oder als Säureadditionssalze isoliert werden.

10. Arzneimittel zur Bekämpfung von Schmerzzuständen, dadurch gekennzeichnet, daß es als Wirkstoff eine oder mehrere Verbindungen gemäß den Ansprüchen 1 bis 8 enthält.

**Claims**

1. Tautomeric arylaminoimidazoline derivatives of the general formulae

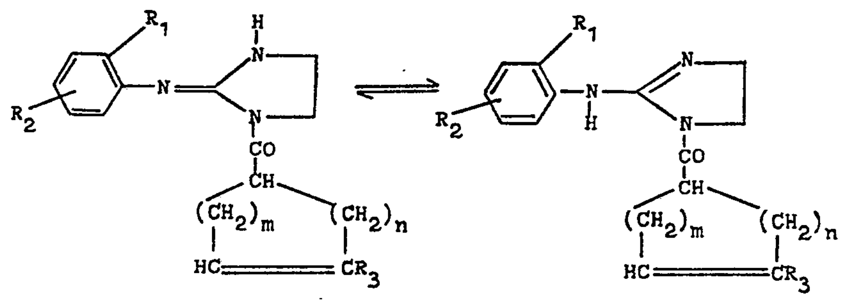

(I)

in which $R_1$ and $R_2$ denote halogen atoms or an alkyl group with 1 to 4 C atoms, and $R_3$ denotes a hydrogen atom or an alkyl radical with 1 to 4 C atoms and n and m each represent an integer from 1 to 3, with the proviso that the sum n+m is an integer from 2 to 5 inclusive, and the acid addition salts of these compounds.

2. Arylaminoimidazoline derivative according to Patent Claim 1, which is 1-(cyclohex-3″-en-1″-oyl)-2-(2′,6′-dichlorophenylamino)-2-imidazoline.

3. Arylaminoimidazoline derivative according to Patent Claim 1, which is 1-(cyclohex-3″-en-1″-oyl)-2-(2′-chloro-6′-methyl-phenylamino)-2-imidazoline.

4. Arylaminoimidazoline derivative according to Patent Claim 1, which is 1-(cyclohex-3″-en-1″-oyl)-2-(2′,3′-dichlorophenylamino)-2-imidazoline.

5. Arylaminoimidazoline derivative according to Patent Claim 1, which is 1-(cyclohex-3″-en-1″-oyl)-2-(2′-methyl-3′-chlorophenylamino)-2-imidazoline.

6. Arylaminoimidazoline derivative according to Patent Claim 1, which is 1-(4″-methyl-cyclohex-3″-en-1″-oyl)-2-(2′,6′-dichlorophenylamino)-2-imidazoline.

7. Arylaminoimidazoline derivative according to Patent Claim 1, which is 1-(cyclohept-4″-en-1″-oyl)-2-(2′,6′-dichlorophenylamino)-2-imidazoline.

8. Arylaminoimidazoline derivative according to Patent Claim 1, which is 1-(cyclopent-3″-en-1″-oyl)-2-(2′,6′-dichlorophenylamino)-2-imidazoline.

9. Process for the preparation of compounds according to Patent Claims 1 to 8, characterised in that

a) an aniline derivative of the general formula

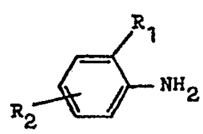

(II)

in which $R_1$ and $R_2$ are as defined in Patent Claim 1, is reacted with a 1-acyl-imidazolidin-2-one of the general formula

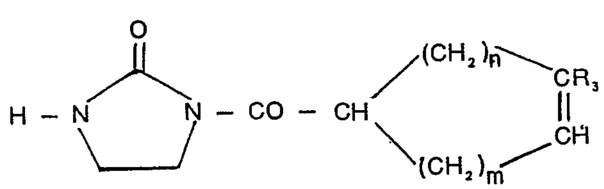

(III)

in which $R_3$, n and m are as defined in Patent Claim 1, in the presence of at least 2 equivalents of phosphorus oxychloride and, after separating off the excess phosphorus oxychloride, the reaction product formed is subjected to mild hydrolysis, or

b) 2-arylamino-2-imidazolines of the general formulae

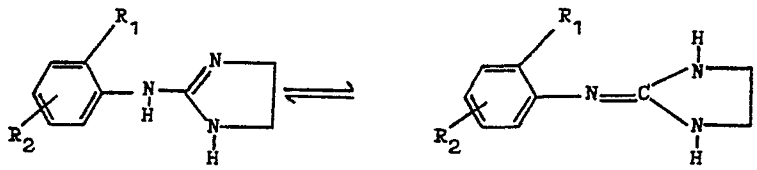

(IV)

in which $R_1$ and $R_2$ are as defined in Patent Claim 1, are reacted with a carboxylic acid derivative of the formula

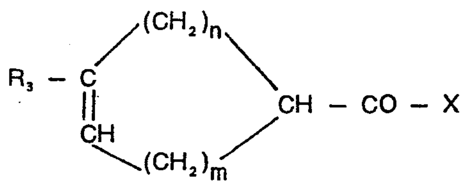

(V)

in which $R_3$, n and m as defined in Patent Claim 1 and X represents a radical of a hetero-aromatic, five-membered ring which contains at least two nitrogen atoms, is optionally fused to a benzene nucleus, and is bonded to the carbonyl group via a nitrogen atom, a radical of the formula

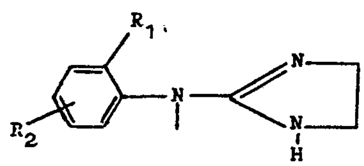

(VI)

in which $R_1$ and $R_2$ are as defined in Patent Claim 1 or an acid radical of the formula

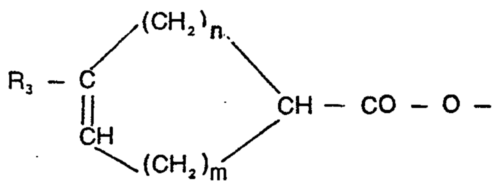

(VII)

in which $R_3$, n and m are as defined in Patent Claim 1, and the resulting compounds of the formula I are isolated as free bases or as acid addition salts.

10. Medicament to control conditions of pain, characterised in that it contains, as the active compound, one or more compounds according to Claims 1 to 8.

**Revendications**

1. Dérivés tautomères d'arylaminoimidazolines de formules générales:

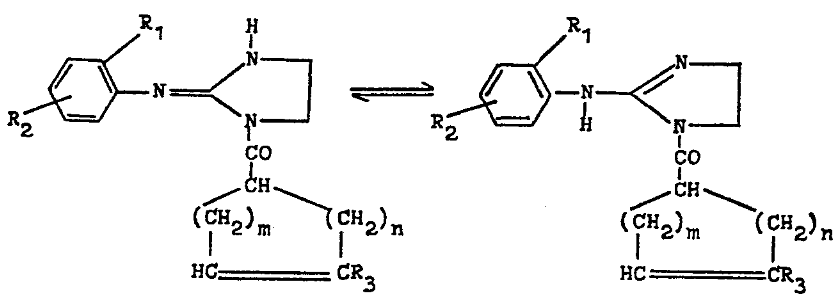

(I)

où $R_1$ et $R_2$ représentent des atomes d'halogène ou un groupe alcoyle ayant de 1 à 4 atomes de carbone et $R_3$ représente un atome d'hydrogène ou un radical alcoyle ayant de 1 à 4 atomes de carbone et n et m représentent chacun un nombre entier de 1 à 3, à condition que la somme de n+m soit un nombre de 2 à 5 inclusivement, et les sels d'addition avec un acide de ces composés.

2. Dérivé d'arylaminoimidazoline suivant la revendication 1, constitué par la 1-(cyclohex-3''-én-1''-oyl)-2-(2',6'-dichlorophénylamino)-2-imidazoline.

3. Dérivé d'arylaminoimidazoline suivant la revendication 1, constitué par la 1-(cyclohex-3''-én-1''-oyl)-2-(2'-chloro-6'-méthyl-phénylamino)-2-imidazoline.

4. Dérivé d'arylaminoimidazoline suivant la revendication 1, constitué par la 1-(cyclohex-3''-én-1''-oyl)-2-(2',3'-dichlorophénylamino)-2-imidazoline.

5. Dérivé d'arylaminoimidazoline suivant la revendication 1, constitué par la 1-(cyclohex-3''-én-1''-oyl)-2-(2'-méthyl-3'-chlorophényl-amino)-2-imidazoline.

6. Dérivé d'arylaminoimidazoline suivant la revendication 1, constitué par la 1-(4''-méthyl-cyclohex-3''-én-1''-oyl)-2-(2',6'-dichlorophénylamino)-2-imidazoline.

7. Dérivé d'arylaminoimidazoline suivant la revendication 1, constitué par la 1-(cyclohept-4''-én-1''-oyl)-2-(2',6'-dichlorophénylamino)-2-imidazoline.

8. Dérivé d'arylaminoimidazoline suivant la revendication 1, constitué par la 1-(cyclopent-3''-én-1''-oyl)-2-(2',6'-dichlorophénylamino)-2-imidazoline.

9. Procédé de préparation de composés suivant les revendications 1 à 8, caractérisé en ce que:
a) on fait réagir un dérivé d'aniline de formule générale:

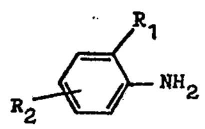

(II)

où $R_1$ et $R_2$ sont tels que définis dans la revendication 1, avec une 1-acylimidazolidin-2-one de formule générale:

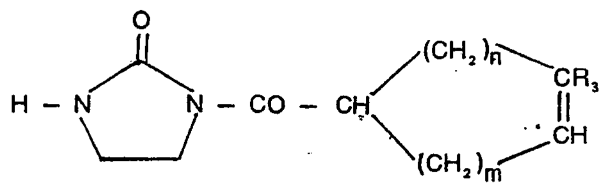

(III)

où $R_3$, n et m sont tels que définis dans la revendication 1, en présence d'au moins deux équivalents d'oxychlorure de phosphore, et en ce qu'on soumet le produit réactionnel obtenu à une hydrolyse douce, après séparation de l'oxychlorure de phosphore en excés ou
b) on fait réagir des 2-arylamino-2-imidazolines de formules générales:

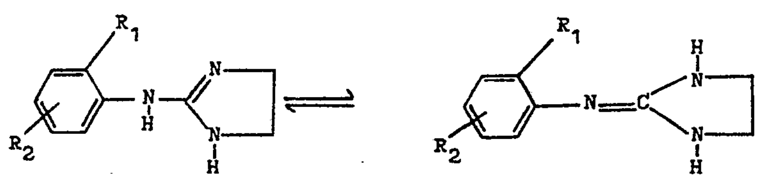

(IV)

où $R_1$ et $R_2$ sont tels que définis dans la revendication 1, avec un dérivé d'acide carboxylique de formule:

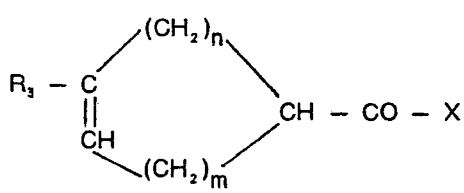

(V)

où $R_3$, n et m ont les significations indiquées dans la revendication 1 et X représente un radical de cycle hétéro-aromatique à 5 chaînons comportant au moins deux atomes d'azote, pouvant être éventuellement condensé avec un noyau benzénique, lié au groupe carbonyle par l'intermédiaire d'un atome d'azote, un radical de formule:

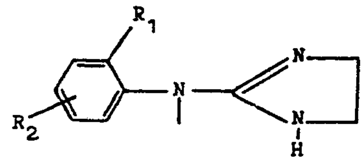

(VI)

où $R_1$ et $R_2$ ont les significations indiquées dans la revendication 1 ou un radical d'acide de formule:

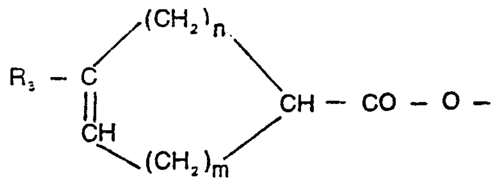

(VII)

où $R_3$, n et m sont tels ue définis dans la revendication 1 et en ce qu'on isole les composés obtenus de formule I sous forme de bases libres ou sous forme de sels d'addition avec un acide.

10. Médicament pour combattre les états douloureux, caractérisé en ce qu'il contient, comme substance active, un ou plusieurs composés suivant les revendications 1 à 8.